Europäisches Patentamt

European Patent Office

Office européen des brevets

(19)

(11) Veröffentlichungsnummer: **0 175 840**
**B1**

(12) **EUROPÄISCHE PATENTSCHRIFT**

(45) Veröffentlichungstag der Patentschrift:
05.10.88

(51) Int. Cl.⁴: **C 07 B 55/00,** C 07 C 149/247,
C 07 C 103/48, C 07 C 103/46,
C 07 D 209/20

(21) Anmeldenummer: 85106048.3

(22) Anmeldetag: 17.05.85

(54) **Verfahren zur Racemisierung von N-Acetyl-D(L)-alpha-aminocarbonsäuren.**

(30) Priorität: 25.09.84 DE 3435095

(43) Veröffentlichungstag der Anmeldung:
02.04.86 Patentblatt 86/14

(45) Bekanntmachung des Hinweises auf die Patenterteilung:
05.10.88 Patentblatt 88/40

(84) Benannte Vertragsstaaten:
AT BE CH DE FR GB IT LI LU NL SE

(56) Entgegenhaltungen:
EP - A - 0 137 371
DE - A - 1 964 420
DE - A - 2 446 320
FR - A - 2 074 022

(73) Patentinhaber: Degussa Aktiengesellschaft,
Weissfrauenstrasse 9, D-6000 Frankfurt am Main 1 (DE)

(72) Erfinder: Karrenbauer, Michael, Dr., Hegaustrasse 1,
D-7761 Moos-Bankholzen (DE)
Erfinder: Kleemann, Axel, Dr., Bornweg 36,
D-6052 Mühlheim (DE)

## Beschreibung

Die Erfindung betrifft ein Verfahren zur Racemisierung von N-Acetyl-D(L)-α-aminocarbonsäuren durch Erhitzen ihrer Schmelze auf höhere Temperaturen.

Solche N-Acetyl-D(L)-α-aminocarbonsäuren fallen in Form ihrer Salze bei der enzymatischen Hydrolyse der Salze von N-Acetyl-D,L-α-aminocarbonsäuren mittels einer L-Aminosäureacylase an. Sie bestehen überwiegend aus N-Acetyl-D-α-aminocarbonsäuren und können daneben noch geringere Mengen an den entsprechenden N-Acetyl-L-α-aminocarbonsäuren enthalten. Sie werden im allgemeinen nach Abtrennung der bei der Hydrolyse gebildeten L-α-Aminocarbonsäure racemisiert und erneut zur enzymatischen Spaltung eingesetzt.

Es ist bereits bekannt, N-Acetyl-D(L)-α-aminocarbonsäuren durch Erhitzen ihrer Schmelze auf höhere Temperaturen zu racemisieren (vgl. DE-A-2446320). Zur Erzielung einer vollständigen Racemisierung sind jedoch relativ lange Verweilzeiten erforderlich, was zu einer starken Verfärbung und zur Bildung erheblicher Mengen an Zersetzungsprodukten führt.

Das erfindungsgemässe Verfahren ist nun dadurch gekennzeichnet, dass man der Schmelze 0,1 bis 2 Gewichtsprozent, bezogen auf die eingesetzte N-Acetyl-D(L)-α-aminocarbonsäure, an Essigsäureanhydrid zusetzt und sie dann für eine Verweilzeit $\tau$ (in Minuten) auf eine Temperatur zwischen 115 und 210 °C erhitzt, wobei die Schmelztemperatur der N-Acetyl-D(L)-α-aminocarbonsäure die Untergrenze der Erhitzungstemperatur T (in °C) darstellt und Erhitzungstemperatur und Verweilzeit durch die Beziehung

$$T = \ln \left(e^{-50\tau + 215} + e^{-5/3\tau + 155}\right)$$

verknüpft sind, und die Schmelze nach Ablauf der Verweilzeit mit einer wässrigen Alkalimetallhydroxid- oder Ammoniaklösung abschreckt.

Vorzugsweise wird das Essigsäureanhydrid der Schmelze in einer Menge zwischen 0,5 und 1 Gewichtsprozent zugesetzt. Als Erhitzungstemperatur der mit dem Essigsäureanhydrid versetzten Schmelze wird vorteilhafterweise eine Temperatur gewählt, die 5 bis 10 °C über der Schmelztemperatur der jeweiligen N-Acetyl-D(L)-α-aminocarbonsäure liegt. Das Aufschmelzen der N-Acetyl-D(L)-α-aminocarbonsäure und das Erhitzen der Schmelze erfolgt vorzugsweise unter Stickstoff. Das Aufschmelzen und das Erhitzen der Schmelze können jedoch auch ohne Schutzgas oder im Vakuum vorgenommen werden.

Das erfindungsgemässe Verfahren erfordert überraschenderweise zu einer praktisch vollständigen Racemisierung nur relativ kurze Verweilzeiten, die beträchtlich unter denen liegen, die beim Erhitzen der Schmelze in Abwesenheit von Essigsäureanhydrid angewandt werden müssen. Dadurch werden Verfärbungen und die Bildung von Zersetzungsprodukten weitgehend vermieden.

Die beim erfindungsgemässen Verfahren einzusetzende N-Acetyl-D(L)-α-aminocarbonsäure wird zweckmässigerweise so gewonnen, dass man die bei der enzymatischen Hydrolyse gebildete Lösung über einen stark sauren Ionenaustauscher schickt, der die enthaltenen Kationen und die L-α-Aminocarbonsäure adsorbiert. Die aus dem Ionenaustauscher austretende Lösung besteht dann praktisch nur noch aus Wasser, Essigsäure und der N-Acetyl-D(L)-α-aminocarbonsäure. Sie wird unter Einhaltung möglichst geringer Verweilzeiten, beispielsweise durch eine Kombination aus einem Fallfilmverdampfer und einem Dünnschichtverdampfer mit Feststoffaustrag, zur Trockne eingedampft und die hinterbleibende N-Acetyl-D(L)-α-aminocarbonsäure wird in dieser Form der erfindungsgemässen Behandlung unterworfen.

Auch die zum Aufschmelzen der N-Acetyl-D(L)-α-aminocarbonsäure erforderliche Verweilzeit soll zweckmässigerweise möglichst kurz gehalten werden. Nimmt man das Aufschmelzen in einem beheizten Extruder vor, so reicht für das vollständige Aufschmelzen im allgemeinen eine Verweilzeit von weniger als einer Minute. In diesem Fall kann der Extruder die Schmelze in ein beheiztes Reaktionsrohr fördern, wo zu Beginn der Verweilzeitstrecke eine entsprechend ausgelegte Pumpe kontinuierlich die erforderliche Menge an Essigsäureanhydrid über ein Mischsystem zudosiert.

Nach Ablauf der für die Racemisierung erforderlichen Verweilzeit, gerechnet ab der Zugabe des Essigsäureanhydrids, wird die Schmelze mit einer wässrigen Alkalimetallhydroxid- oder Ammoniaklösung abgeschreckt. Dabei ist es zweckmässig, gleich die für die anschliessende erneute enzymatische Spaltung erforderliche Substratkonzentration einzustellen.

Die Erfindung wird durch die nachfolgenden Beispiele näher erläutert. Alle Prozentangaben bedeuten, wenn nicht anders angegeben, Gewichtsprozente.

Die eingesetzten N-Acetyl-D(L)-α-aminocarbonsäuren und die racemisierten Proben werden jeweils auf ihren spezifischen Drehwert $[\alpha]_D^{20}$ in Grad · cm³/dm · g untersucht.

Beispiel 1

10 g (0,053 mol) N-Acetyl-D(L)-methionin (Schmelztemperatur ∼108 °C) wurden bei 118 °C unter Stickstoff in einem zwangsfördernden, zusatzbeheizten Extruder innerhalb einer Verweilzeit von einer Minute aufgeschmolzen.

Die Schmelze wurde mit 0,1 g Essigsäureanhydrid versetzt und anschliessend noch 21 Minuten lang bei 120 °C unter Stickstoff gerührt, um dann mit ca. 80 ml einer 1 prozentigen wässrigen Ammoniaklösung abgeschreckt zu werden, wobei die Temperatur auf ca. 40 °C absank. Die Lösung wurde durch Zugabe von weiterem wässrigem Ammoniak auf pH 7 und durch Zudosierung von Wasser auf die bei der enzymatischen Reaktion einzusetzende Substratkonzentration (0,6 molar) eingestellt.

Der durch Hochdruckflüssigkeitschromatographie bestimmte Gehalt an N-Acetyl-D,L-methionin betrug 99,1% der Theorie.

$[\alpha]_D^{20}$ vor der Racemisierung: +17,85° (c = 4 in Wasser)

$[\alpha]_D^{20}$ nach der Racemisierung: ±0° (c = 4 in Wasser) Beispiel 2

Das Beispiel 1 wurde wiederholt mit dem Unterschied, dass die bei 118 °C erhaltene Schmelze anschliessend im Vakuum 18 Minuten lang auf 125 °C erhitzt wurde.

Der durch Hochdruckflüssigkeitschromatographie bestimmte Gehalt an N-Acetyl-D,L-methionin betrug 97,3% der Theorie.

$[\alpha]_D^{20}$ vor der Racemisierung: +17,85° (c = 4 in Wasser)

$[\alpha]_D^{20}$ nach der Racemisierung: ±0° (c = 4 in Wasser)

**Beispiel 3**

Das Beispiel 1 wurde wiederholt mit dem Unterschied, dass das N-Acetyl-D(L)-methionin in einem zusatzbeheizten Extruder in kontinuierlicher Fahrweise bei 160 °C ohne Schutzgas innerhalb einer Verweilzeit von 30 Sekunden aufgeschmolzen wurde. Die Schmelze wurde mit 0,05 g Essigsäureanhydrid versetzt und noch 1,5 Minuten lang in einem nachgeschalteten Reaktionsrohr auf 160 °C erhitzt.

Der durch Hochdruckflüssigkeitschromatographie bestimmte Gehalt an N-Acetyl-D,L-methionin betrug 98,5% der Theorie.

$[\alpha]_D^{20}$ vor der Racemisierung: +17,85° (c = 4 in Wasser)

$[\alpha]_D^{20}$ nach der Racemisierung: ±0° (c = 4 in Wasser)

**Beispiel 4**

Das Beispiel 1 wurde wiederholt mit dem Unterschied, dass das N-Acetyl-D(L)-methionin bei 115 °C aufgeschmolzen und die mit dem Essigsäureanhydrid versetzte Schmelze noch 24 Minuten lang auf 115 °C erhitzt wurde.

Der durch Hochdruckflüssigkeitschromatographie bestimmte Gehalt an N-Acetyl-D,L-methionin betrug 99% der Theorie.

$[\alpha]_D^{20}$ vor der Racemisierung: +17,85° (c = 4 in Wasser)

$[\alpha]_D^{20}$ nach der Racemisierung: ±0° (c = 4 in Wasser)

**Beispiel 5**

Das Beispiel 1 wurde wiederholt mit dem Unterschied, dass das N-Acetyl-D(L)-methionin in einem zwangsfördernden, zusatzbeheizten Extruder in kontinuierlicher Fahrweise bei 200 °C innerhalb einer Verweilzeit von 45 Sekunden aufgeschmolzen wurde. Die Schmelze wurde mit 0,05 g Essigsäureanhydrid versetzt und noch 18 Sekunden lang in einem nachgeschalteten Reaktionsrohr auf 200 °C erhitzt.

Der durch Hochdruckflüssigkeitschromatographie bestimmte Gehalt an N-Acetyl-D,L-methionin betrug 96,2% der Theorie.

$[\alpha]_D^{20}$ vor der Racemisierung: +17,85° (c = 4 in Wasser)

$[\alpha]_D^{20}$ nach der Racemisierung: ±0° (c = 4 in Wasser)

**Vergleichsversuch 1**

Das Beispiel 1 wurde wiederholt mit dem Unterschied, dass die bei 118 °C erhaltene Schmelze mit 0,5 g Essigsäureanhydrid versetzt und anschliessend noch 18 Minuten lang auf 125 °C erhitzt wurde.

Der durch Hochdruckflüssigkeitschromatographie bestimmte Gehalt an N-Acetyl-D,L-methionin betrug 93% der Theorie.

$[\alpha]_D^{20}$ vor der Racemisierung: +17,85° (c = 4 in Wasser)

$[\alpha]_D^{20}$ nach der Racemisierung: ±0° (c = 4 in Wasser)

**Beispiel 6**

10 g (0,076 mol) N-Acetyl-D(L)-alanin (Schmelztemperatur ~130 °C) wurden bei 135 °C unter Stickstoff in einem zwangsfördernden, zusatzbeheizten Extruder innerhalb einer Verweilzeit von einer Minute aufgeschmolzen.

Die Schmelze wurde mit 0,1 g Essigsäureanhydrid versetzt und anschliessend noch 12 Minuten lang bei 135 °C unter Stickstoff gerührt, um dann mit 50 ml einer 4 prozentigen Natronlauge abgeschreckt zu werden. Die weitere Aufarbeitung erfolgte analog Beispiel 1.

Der durch Hochdruckflüssigkeitschromatographie bestimmte Gehalt an N-Acetyl-D,L-alanin betrug 99,2% der Theorie.

$[\alpha]_D^{20}$ vor der Racemisierung: +65,4° (c = 2 in Wasser)

$[\alpha]_D^{20}$ nach der Racemisierung: +0,1° (c = 2 in Wasser)

**Beispiel 7**

Das Beispiel 6 wurde wiederholt mit dem Unterschied, dass das N-Acetyl-D(L)-alanin in einem zusatzbeheizten Extruder in kontinuierlicher Fahrweise bei 170 °C innerhalb einer Verweilzeit von 50 Sekunden aufgeschmolzen wurde. Die Schmelze wurde mit 0,05 g Essigsäureanhydrid versetzt und noch ca. eine Minute lang in einem nachgeschalteten Reaktionsrohr auf 170 °C erhitzt.

Der durch Hochdruckflüssigkeitschromatographie bestimmte Gehalt an N-Acetyl-D,L-alanin betrug 98,7% der Theorie.

$[\alpha]_D^{20}$ vor der Racemisierung: +65,4° (c = 2 in Wasser)

$[\alpha]_D^{20}$ nach der Racemisierung: +0,4° (c = 2 in Wasser)

**Vergleichsversuch 2**

Das Beispiel 6 wurde wiederholt mit dem Unterschied, dass die bei 135 °C erhaltene Schmelze mit 0,5 g Essigsäureanhydrid versetzt und anschliessend noch 12 Minuten lang bei 135 °C gerührt wurde.

Der durch Hochdruckflüssigkeitschromatographie bestimmte Gehalt an N-Acetyl-D,L-alanin betrug 92,7% der Theorie.

$[\alpha]_D^{20}$ vor der Racemisierung: +65,4° (c = 2 in Wasser)

$[\alpha]_D^{20}$ nach der Racemisierung: ±0° (c = 2 in Wasser)

**Beispiel 8**

20 g (0,097 mol) N-Acetyl-D(L)-phenylalanin (Schmelztemperatur ~167 °C) wurden bei 175 °C unter Stickstoff in einem zusatzbeheizten Extruder innerhalb einer Verweilzeit von 45 Sekunden aufgeschmolzen.

Die Schmelze wurde mit 0,1 g Essigsäureanhydrid versetzt und anschliessend noch 55 Sekunden lang in einem nachgeschalteten Reaktionsrohr auf 170 °C gehalten, um dann mit 90 ml einer 4 prozentigen Natronlauge abgeschreckt zu werden. Die Lösung wurde durch Zugabe von weiterer Natronlauge auf pH 7 und durch Zudosierung von Wasser auf die bei der enzymatischen Spaltung einzusetzende Substratkonzentration (0,4 molar) eingestellt.

Der durch Hochdruckflüssigkeitschromatographie bestimmte Gehalt an N-Acetyl-D,L-phenylalanin betrug 99% der Theorie.

$[\alpha]_D^{20}$ vor der Racemisierung: −46,85° (c = 2 in Ethanol)

$[\alpha]_D^{20}$ nach der Racemisierung: ±0° (c = 2 in Ethanol)

**Beispiel 9**

Das Beispiel 8 wurde wiederholt mit dem Unterschied, dass die bei 175 °C erhaltene Schmelze mit 0,2 g Essigsäureanhydrid versetzt und anschliessend noch 50 Sekunden lang auf 175 °C erhitzt wurde.

Der durch Hochdruckflüssigkeitschromatographie bestimmte Gehalt an N-Acetyl-D,L-phenylalanin betrug 98,5% der Theorie.

$[\alpha]_D^{20}$ vor der Racemisierung: −46,85° (c = 2 in Ethanol)

$[\alpha]_D^{20}$ nach der Racemisierung: ±0° (c = 2 in Ethanol)

**Beispiel 10**

Das Beispiel 8 wurde wiederholt mit dem Unterschied, dass die bei 175 °C erhaltene Schmelze mit 0,4 g Essigsäureanhydrid versetzt und anschliessend noch 50 Sekunden lang auf 170 °C gehalten wurde.

Der Hochdruckflüssigkeitschromatographie bestimmte Gehalt an N-Acetyl-D,L-phenylalanin betrug 97,5% der Theorie.

$[\alpha]_D^{20}$ vor der Racemisierung: −46,85° (c = 2 in Ethanol)

$[\alpha]_D^{20}$ nach der Racemisierung: ±0° (c = 2 in Ethanol)

**Vergleichsversuch 3**

Das Beispiel 10 wurde wiederholt mit dem Unterschied, dass die Schmelze mit 1,0 g Essigsäureanhydrid versetzt wurde.

Der durch Hochdruckflüssigkeitschromatographie bestimmte Gehalt an N-Acetyl-D,L-phenylalanin betrug 92,2% der Theorie.

$[\alpha]_D^{20}$ vor der Racemisierung: −46,85° (c = 2 in Ethanol)

$[\alpha]_D^{20}$ nach der Racemisierung: ±0° (c = 2 in Ethanol)

**Beispiel 11**

10 g (0,041 mol) N-Acetyl-D(L)-tryptophan (Schmelztemperatur ~193 °C) wurden bei 205 °C unter Stickstoff in einem zusatzbeheizten Extruder innerhalb einer Verweilzeit von 45 Sekunden aufgeschmolzen.

Die Schmelze wurde mit 0,05 g Essigsäureanhydrid versetzt und anschliessend noch 12 Sekunden lang in einem nachgeschalteten Reaktionsrohr auf 205 °C gehalten, um anschliessend mit 150 ml einer 1 prozentigen Natronlauge abgeschreckt und auf die bei der enzymatischen Spaltung einzusetzende Substratkonzentration (0,2 molar) eingestellt zu werden.

Das NMR-Spektrum war identisch mit dem des Ausgangsmaterials, was einen Gehalt an N-Acetyl-D,L-tryptophan von mindestens 95% der Theorie bedeutet.

$[\alpha]_D^{20}$ vor der Racemisierung: −23,0° (c = 5 in Methanol)

$[\alpha]_D^{20}$ nach der Racemisierung: −0,02° (c = 5 in Methanol)

**Beispiel 12**

10 g (0,063 mol) N-Acetyl-D(L)-valin (Schmelztemperatur ~162 °C) wurden bei 170 °C unter Stickstoff in einem zusatzbeheizten Extruder innerhalb einer Verweilzeit von 40 Sekunden aufgeschmolzen.

Die Schmelze wurde mit 0,05 g Essigsäureanhydrid versetzt und anschliessend noch 55 Sekunden lang in einem nachgeschalteten Reaktionsrohr auf 170 °C gehalten, um anschliessend mit 50 ml einer 4 prozentigen Natronlauge abgeschreckt zu werden. Die weitere Aufarbeitung erfolgte analog Beispiel 1.

**Patentansprüche**

1. Verfahren zur Racemisierung von N-Acetyl-D(L)-α-aminocarbonsäuren durch Erhitzen ihrer Schmelze auf höhere Temperaturen, dadurch gekennzeichnet, dass man der Schmelze 0,1 bis 2 Gewichtsprozent, bezogen auf die eingesetzte N-Acetyl-D(L)-α-aminocarbonsäure, an Essigsäureanhydrid zusetzt und sie dann für eine Verweilzeit $\tau$ (in Minuten) auf eine Temperatur zwischen 115 und 210 °C erhitzt, wobei die Schmelztemperatur der N-Acetyl-D(L)-α-aminocarbonsäure die Untergrenze der Erhitzungstemperatur T (in °C) darstellt und Erhitzungstemperatur und Verweilzeit durch die Beziehung

$$T = \ln\left(e^{-50\tau + 215} + e^{-5/3\tau + 155}\right)$$

verknüpft sind, und die Schmelze nach Ablauf der Verweilzeit mit einer wässrigen Alkalimetallhydroxid- oder Ammoniaklösung abschreckt.

Der durch Hochdruckflüssigkeitschromatographie bestimmte

Gehalt an N-Acetyl-D,L-valin betrug 99,1% der Theorie.

$[\alpha]_D^{20}$ vor der Racemisierung: +17,7° (c = 4 in Wasser)

$[\alpha]_D^{20}$ nach der Racemisierung: +0,6° (c = 4 in Wasser)

2. Verfahren nach Anspruch 1, dadurch gekennzeichnet, dass man der Schmelze 0,5 bis 1 Gewichtsprozent an Essigsäureanhydrid zusetzt.

3. Verfahren nach Anspruch 1 oder 2, dadurch gekennzeichnet, dass man das Erhitzen der Schmelze bei einer Temperatur vornimmt, die 5 bis 10 °C über der Schmelztemperatur der N-Acetyl-D(L)-α-aminocarbonsäure liegt.

4. Verfahren nach einem der Ansprüche 1 bis 3, dadurch gekennzeichnet, dass man das Aufschmelzen der N-Acetyl-D(L)-α-aminocarbonsäure und das Erhitzen der Schmelze unter Stickstoff vornimmt.

**Revendications**

1. Procédé de racémisation d'acides N-acétyl D(L) α-amino carboxyliques par chauffage de leur produit de fusion à des températures élevées, caractérisé en ce que l'on additionne le produit de fusion avec 0,1 à 2% en poids – rapporté à l'acide N-acétyl D(L) α-amino carboxylique mis en œuvre – d'anhydride acétique et les chauffe ensuite pendant un temps de séjour τ (en minutes) à une température comprise entre 115 et 210 °C, pour laquelle la température de fusion de l'acide N-acétyl D(L) α-amino carboxylique représente la limite inférieure de la température de chauffage T (en degré C) et la température de chauffage et le temps de séjour sont reliés par la relation:

$$T = \ln (e^{-50\tau + 215} + e^{-5/3\tau + 155})$$

et le produit de fusion après écoulement du temps de séjour est refroidi avec une solution aqueuse d'un hydroxyde de métal alcalin ou d'ammoniaque.

2. Procédé selon la revendication 1, caractérisé en ce que le produit de fusion est additionné de 0,5 à 1% en poids d'anhydride acétique.

3. Procédé selon la revendication 1 ou 2, caractérisé en ce que l'on effectue le chauffage du produit de fusion à une température qui se situe de 5 à 10 °C au dessus de la température de fusion.

4. Procédé selon l'une des revendications 1 à 3, caractérisé en ce que l'on effectue la fusion de l'acide N-acétyl D(L) α-amino carboxylique et le chauffage du produit de fusion sous une atmosphère d'azote.

**Claims**

1. Process for the racemization of N-acetyl-D(L)-α-aminocarboxylic acids by heating melts thereof to fairly high temperatures, characterized in that 0.1 to 2 per cent by weight, relative to the N-acetyl-D(L)-α-aminocarboxylic acid employed, of acetic anhydride is added to the melt, and the melt is then heated at a temperature between 115 and 210 °C for a dwell time τ (in minutes), the melting point of the N-acetyl-D(L)-α-aminocarboxylic acid constituting the lower limit of the heating temperature T (in °C) and the heating temperature and the dwell time being linked by the relationship

$$T = \ln (e^{-50\tau + 215} + e^{-5/3\tau + 155})$$

and, after the expiry of the dwell time, the melt is chilled with an aqueous solution of an alkali metal hydroxide or ammonia.

2. Process according to Claim 1, characterized in that 0.5 to 1 per cent by weight of acetic anhydride is added to the melt.

3. Process according to Claim 1 or 2, characterized in that the heating of the melt is carried out at a temperature which is 5 to 10 °C above the melting point of the N-acetyl-D(L)-α-aminocarboxylic acid.

4. Process according to one of Claims 1 to 3, characterized in that the melting of the N-acetyl-D(L)-α-aminocarboxylic acid and the heating of the melt are carried out under nitrogen.